# EUROPEAN PATENT APPLICATION

(11) **EP 1 518 526 A1**
(43) Date of publication of application: **30.03.2005**
(21) Application number: 02733580.1
(22) Date of filing: 17.05.2002
(51) Int. Cl.: A61F 13/15, A61F 13/505

(54) **BIODEGRADABLE, ENVIRONMENTALLY-FRIENDLY PANTS DIAPER**

(71) Applicant: Flores Gonzales, Estela Concepcion, Santiago de Querétaro, Querétaro 76020 (MX)
(72) Inventor: Flores Gonzales, Estela Concepcion, Santiago de Querétaro, Querétaro 76020 (MX)
(74) Representative: Abitz, Walter, Dr.-Ing.
(86) International application number: PCT/MX2002/000041
(87) International publication number: WO 2003/096946

(57) **Abstract**

**This invention is a 100% biodegradable, disposable diaper-underpants.**
It is made with a waterproof, recyclable and reusable plastic and fabrics diaper or underpants, in the inside it has a paper disposable pad, made with natural and vegetable materials, being comfortable, absorbing, hygienic and biodegradable
If the first one is kept in good conditions and free of germs, washed and dried as it has to be, with biodegradable detergents, can be used during the entire baby's growth stage and more.
The paper absorbing pad can be separately throw away in disposal rubbish or buried without any contamination of the environment.

## Description

**This invention is a disposable 100% biodegradable diaper - underpants.**

It is a plastic diaper or underpants adhered to another fabric underpants both recyclable and re-usable. Inside they hold a paper absorbing pad completely biodegradable made with natural and vegetable materials.
I do introduce three models for different occasions :
- **Thick diaper maximum protection:** it is a plastic diaper attached to a fabric diaper and two absorbing pads, for daytime or nighttime.
- **Thin diaper :** it is a plastic diaper with stretchable sides and a daytime absorbing pad a little bit shorter.
- **Learning underpants medium protection:** it is a plastic underpants with stretchable sides and daytime and nighttime absorbing pads.

All models can be made in different sizes for children and adults.

Traditional fabric diapers are uncomfortable, unpractical, and very difficult to wash.
Disposable diapers existing in the market today are made with plastic materials, that require many years to decompose, polluting the environment.
The diapers I invented are comfortable, practical, hygienic and do not pollute.

**The outside** is made with a water-proof, re-usable, recyclable plastic and fabric underpants. If after use this keeps dry and clean you could use many times before hand or machine washing using biodegradable detergent and we suggest drying under the sun.
**The inside** or paper pad is natural, comfortable, hygienic and very practical.
It directly receives the organic waste (solid and liquid) and when it gets dirty we change for a new one.
These pads can be thrown away apart in the garbage with the organic wastes. Or they can be buried in the garden or in a field and used as soil fertilizer being 100% biologic including the human organic waste of course, following the natural decomposition process.
I suggest cleaning the baby or adult using dry towels with a cleaning lotion or talc, all biodegradable and not allergic, without scents or artificial colorants.

### - Thick diaper or maximum protection :

**Plastic diaper:** made in satiated plastic PVC (Polyvinyl chloride) smoother and satin the outer cover and with more texture, rough on the inside. White colour, calibre 5/6, recyclable classification number 3.
With almost the same size and shape (form) of a common diaper it has two extra plastic rims in the front and back waist, that turned to the inside. The same happens in the front and back sides, four rims turning to the inside, all these to make the nappy and plastic stronger. The four corners ends can be turned to the inside too.

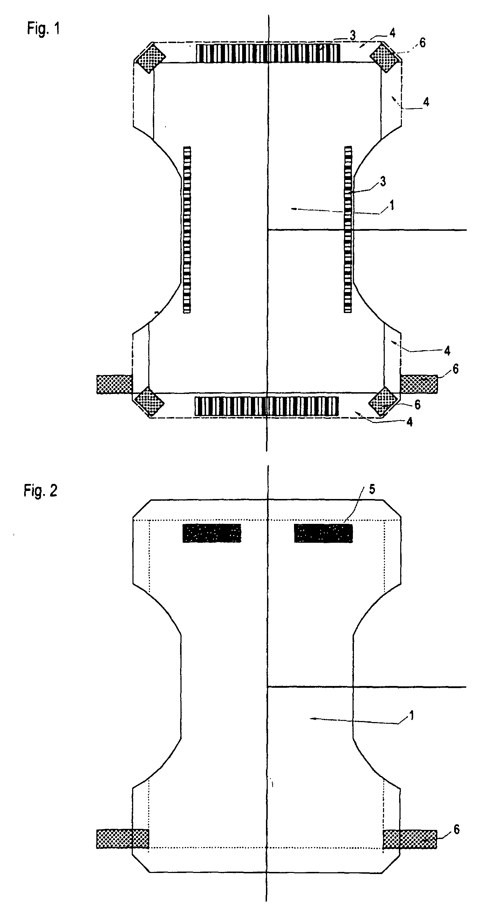

Inside: (figure 1) it has cotton elastics (74 % cotton 26 % rubber latex), in the front and back waist sewn around with straight seam, with 100 % cotton thread.
For a better fitting and protection it has narrow cotton elastics between the legs sewed with zigzag 100 % cotton thread trapunto stitching. Velcro (100 % nylon).

The hook side of the velcro on the four corners sewn around with cotton thread in diagonal position to adhere to the fabric underpants.
Outside : (fig. 2) to the frontal central part slightly underneath the waist two loop side of the velcro strips, in the back side to the same lateral height two hook side of the velcro strips to fit and close the diaper on the baby's body.

**Fabric diaper:** Made with 100 % cotton two flannels pre-washed cloaks.
Plastic diaper same size and shape, but with round corners, it is over stitched with 100 % cotton thin calibre 30/2 thread.

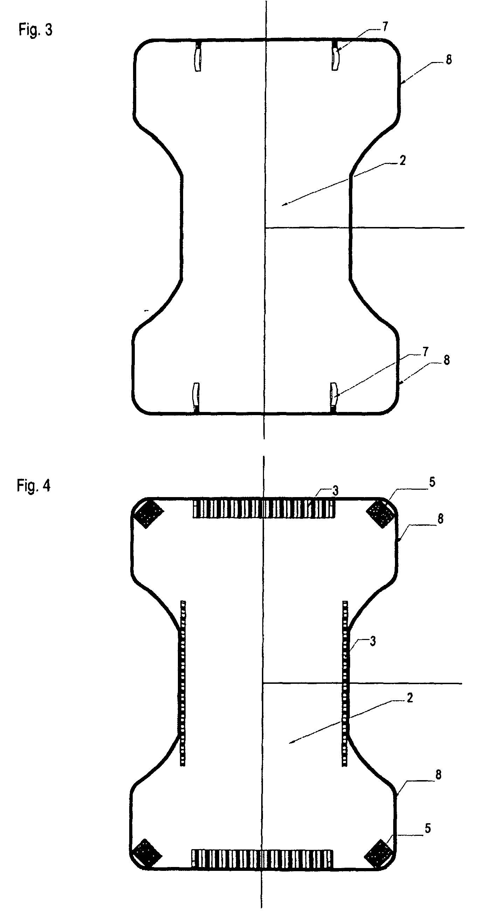

Inside : (fig. 3) In the waist, just where the elastics end, it has rings made with 100 % cotton band; two in the front and two in the back, where the paper pad is tied up.

Outside : (fig. 4) On the front and back of the waist it has cotton elastics sewed around with 100 % cotton thread . Between the legs it has cotton thin elastics sewed with zigzag trap unto stitching and cotton thread. Loop side of the velcro strips on his four corners sewed around with straight line stitching and cotton thread in diagonal position, to be fitted at the plastic diaper.

### - Thin diaper:

Made in plastic PVC white satin smoother and slippered the outer cover, rectangular, with his four rounded corners .

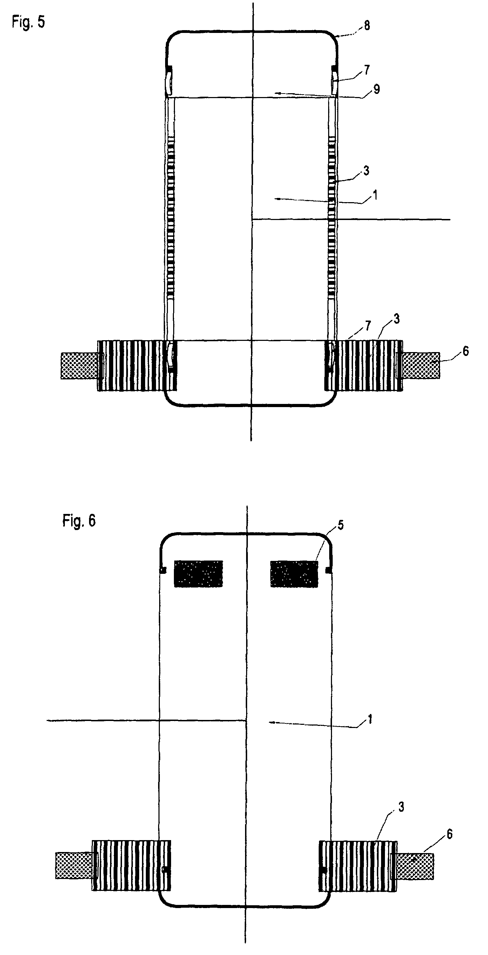

Inside : (fig. 5) Made of 100 % cotton fabric flannel in the front and back waist, sewed on the top only to let the bottom side free for better drying when it is washed and keeping baby's skin away from the plastic and at the top of these sides, to fit the paper pad, it has rings made of cotton, two on the front and two on the back.
Between the legs it has cotton thin elastics sewed with zigzag trapunto stitching, for a good fitting and protection.

Outside : (fig. 6) Just a little bit under the front waist and towards the centre , it has loop side of velcro. On the back side a little bit under the waist, sewed on the sides and coming out from here, wide long cotton elastics to fit the diaper and the end of these, hook side of velcro to close the diaper.

### - Learning underpants medium protection :

For children and adults who can go to bathroom.
Made with shiny white plastic PVC, rectangular shaped, with the smooth side facing out ward.

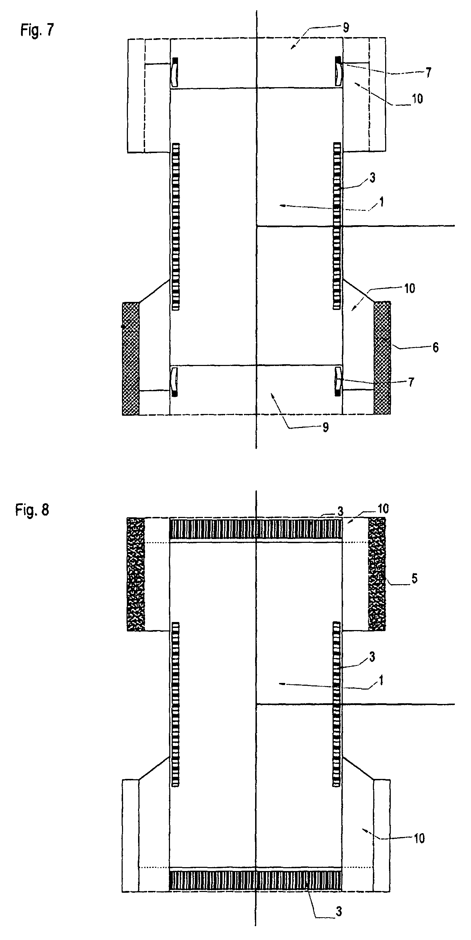

Inside : (fig. 7) Made with 100 % cotton fabric flannel in the front and back waist , only sewed on the top leave bottom side free for better drying when it is washed and keeping the baby's skin away from the plastic and at the top of these sides, to tie the paper pad, it has two rings made of cotton, two on the front and two on the back. Between the legs it has cotton thin elastics sewed with trapunto stitching, for good fitting and protection.
It has two rectangular stretch fabrics (tissue or jersey in cotton-lycra) at the front starting from waist sewed on each side and with rims on the waist sewed around with cotton thread and over stitching. On the back side, starting from the waist sewed on each side it has two trapeze stretch fabrics (tissue or jersey in cotton-lycra) with rims on the waist sewed around with cotton thread and over stitching.
These pieces have hook side of the velcro along the edges.

Outside : (fig. 8) Cotton elastic bands on the front and back waist on the plastic sides. On the edges of rectangular front pieces we have loop side of the velcro strip to open and close the underpants and in any the underpants can be taken off or put on while kept closed.

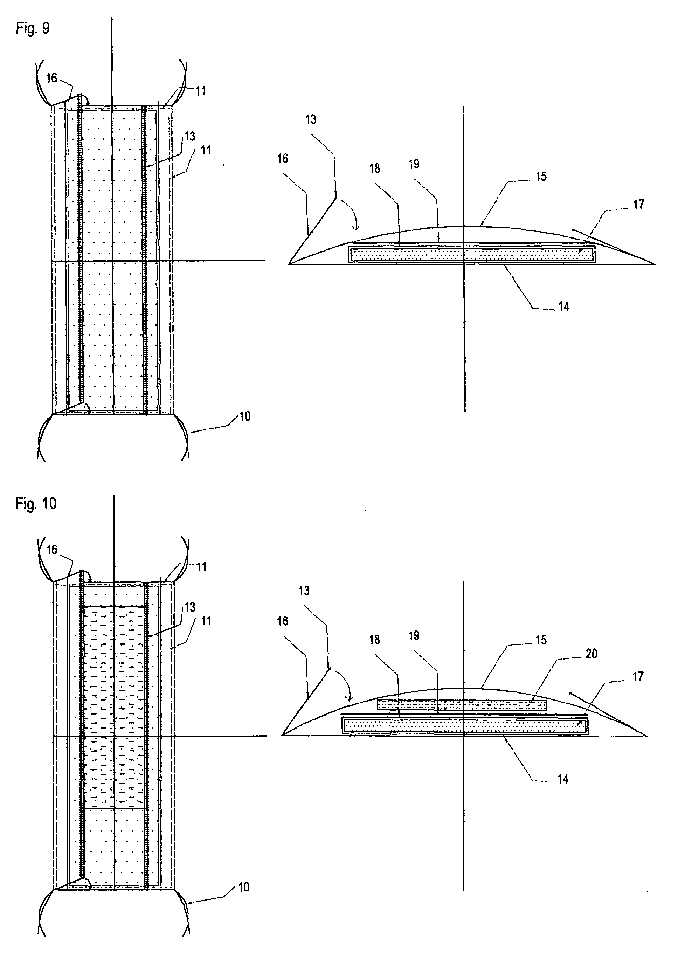

### - Daytime paper absorbing pad : (fig. 9 )

The absorbing pad is rectangular. The outside cover is in contact with the diaper or underpants. It is made with a soft and flexible a fabric type paper, elaborated with virgin cellulose fibers, this paper is waxed with a natural and vegetable and biodegradable wax, it can be double covered or single, (you can also use natural biodegradable cellophane paper or with some biological biodegradable plastic, for example the one produced with the corn plant) and this side has two rims on the edges. Above this we have a cellulose pulp pad mixed with a vegetal absorbent polymer covered with a thin and flexible paper, over this pad goes a thick flexible absorbing paper. And over all this the inside cover that will be in contact with baby skin, it will be made with fabric paper, soft and flexible elaborated with virgin cellulose fibers.
The outside rims will turn above the rest of the pad and we sew in this point with two 100 % cotton calibre 30/2/5(big) and 30/5 (medium) threads, leaving the eight threads ends large at the beginning and at the end of the sewing. It has a straight sewing with stretch thread (74 % cotton- 26 % rubber latex) on the outside waxed cover and on the inside face cotton thread, to build the leak shields. Finally we sew with cotton threads big and medium calibre straight stitching, closing the up and down edges leaving the eight threads ends large at the beginning and at the end of sewing, to tie them up to the others, passing through the plastic or fabric diaper rings.

### - Nighttimes paper absorbing pad (fig. 10)

Like the daytime one with only two differences :
It takes an extra cellulose pulp pad shorter and thinner, it goes centred on the width, but more to the front side to absorb liquids better.
The upper back side sewing on the inside will be in turquoise blue thread to identify the back side. Also the plastic and fabric diapers have a blue thread on the upper back cover on the inside. All this to identify it better and position the absorbent pad correctly.

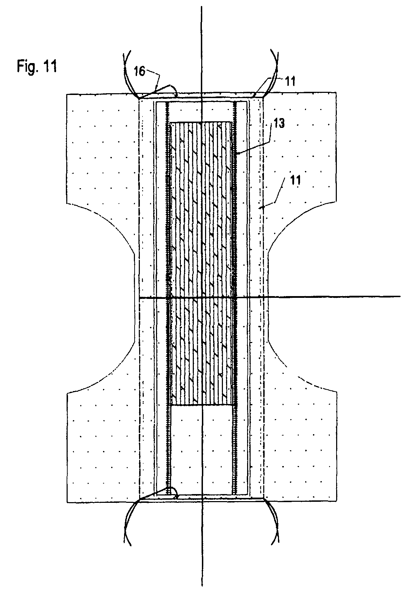

### - Adults thick paper absorbing pad or maximum protection model. (fig. 11)

Same as day and nighttime absorbing pad with few differences. The front and back sides are wider and the inside and outside covers too. So the cellulose pulp pad mixed with the vegetable absorbing polymer and the thick absorbing and flexible paper that goes on the top of this pad. Maintaining the thin form of the area between the legs and the leak shields.

To remove the dirty absorbing pad you have to cut the threads with an safety scissor : with no sharp tip of edge, carefully not to damage the diaper cotton rims.

The percentage of natural materials used to make the diaper is much larger than the percentage of synthetic materials. The synthetic materials used are very long lasting and we don't know how many paper absorbing pads can be used with the same plastic diaper.
All the papers, cellulose pulp, threads, elastics, cotton fabric, wax and absorbing polymer will be ivory or white natural colour, that means there won't be an extra whitewashing process, which is more expensive for material price and that cause pollution using a large quantity of Chlorine and bleaching products that brake the equilibrium of land and water.

World paper industry has reduced his production; in the packing area synthetic materials, as plastic and derivates have replaced paper. So have computers and internet, in the writing documents and graphic publications, only a CD-ROM can hold thousands of text documents.
For example : before a bank client of any bank every month received at home his statement of account written on paper pages also with a paper envelope. Now he is able to know how much money he has any day, any time just pushing some buttons on his personal computer.
Also on the world wide web you have thousands of sites where you can find books, magazines and newspapers for a great virtual public.
The extra cellulose production obtained from a rational and sustained forest and agricultural exploitation can be used to make these diapers.
We stress sustained, because for example small countries as the Nordic ones (Sweden, Finland) have been producing wood and so paper during years and still keep on doing it, thanks to their natural resources care and reforestation.
About agriculture, nowadays for example you can obtain cellulose and so paper from the corn plant.

### Parts

1. White satin plastic PVC Polyvinyl Chloride calibre 5/6 # 3.
2. Double flannel fabric pre washed 100% cotton.
3. Elastic 74% cotton 26% rubber latex.
4. Rim or double plastic edge.
5. Hook strip (Velcro).
6. Loop strip (Velcro).
7. Rings made with 100% cotton band.
8. Over stitched with 100% cotton calibre 30/2 thread.
9. Single flannel fabric pre washed 100% cotton.
10. Stretch tissue fabric or jersey in cotton lycra.
11. Straight seam with 100% cotton thread medium calibre and thick.
12. Large threads ends thick and medium calibre.
13. Seam with 100% cotton thread inside and elastic thread 74% cotton and 26% rubber latex outside.
14. Waxed paper double or single outside cover, with natural wax. (this can be made with natural biodegradable cellophane paper or with some biological biodegradable plastic, for example the one produced with the corn plant).
15. Inside cover made of virgin cellulose fabric-type paper that is soft and flexible.
16. Leak shields that are part of the outside cover.
17. Cellulose pulp pad mixed with a vegetable absorbent polymer.
18. Thin paper covering the cellulose pulp pad with the absorbing polymer.
19. Flexible thick absorbing paper.
20. Extra cellulose pulp pad, for nighttime use.

## Claims

1. **THE BIODEGRADABILITY of my disposal diapers obtained when separating the indispensable qualities of a diaper:**
**Waterproof :** creating a diaper or underwear of plastic and fabric, re-usable and recyclable with material natural and synthetic.
**Absorption :** creating absorbent of disposable paper natural and biodegradable 100 % with paper a type fabric , paper with a natural vegetable wax, (this can be used natural biodegradable cellophane paper or by some biological biodegradable plastic, for example the one produced with the corn plant), polymer wax vegetable absorbing, threads and elastics of cotton , that are united to form a comfortable, practical, hygienic, healthy diaper that will not pollute the environment.

## Amended claims

### Amended claims under Art. 19.1 PCT

1. **THE ECOLOGICAL of my disposal diapers obtained when separating the two indispensable qualities of a diaper:**
**Waterproof:** creating a diaper or underwear of plastic and fabric, waterproof reusable and reciclable
**Absorption:** creating and ecological disposable 100% natural absorbent. Both are put together to get a comfortable, practical, hygienic, healthy diaper that will not pollute the environment.

2. **Diapers ecological underwear** that perfectly fullfil with his function with two indipendent elements ; one disposable ecological absorbent 100 % natural that is put inside of an underwear diaper made of waterproof plastic , syntetic but reusable and recyclable.

3. **Ecological disposable absorbent** with inside cover made of fabric type paper and outside cover made of natural celophan paper or some biological plastic, for example the one produced with the com plant.

4. **Ecological disposable absorbent** with vegetable absorbent polymer inside, that keeps main quantity of fluids.

5. **The ecological absorbents** are tied with 100% cotton and rubber latex and cotton threads. The ends of these threads are fixed to the plastic and fabric underwear diaper. The ecological disposable absorbents are replaced for new ones, while plastic diapers(underwear) are washed or cleaned and finally recicled.
